# EUROPÄISCHE PATENTSCHRIFT

(11) **EP 1 508 317 B1**
(45) Veröffentlichungstag und Bekanntmachung des Hinweises auf die Patenterteilung: **20.09.2006**
(21) Anmeldenummer: 04011381.3
(22) Anmeldetag: 13.05.2004
(51) Int. Cl.: A61F 2/60

(54) **Übergangsadapter**
Connection adapter for exoprotheses
Adapter de raccordement pour des exoprotheses

(30) Priorität: 20.08.2003 DE 10339209
(43) Veröffentlichungstag der Anmeldung: 23.02.2005
(73) Patentinhaber: ESKA Implants GmbH & Co., 23556 Lübeck (DE)
(72) Erfinder: Grundei, Hans, Dr., 23558 Lübeck (DE)
(74) Vertreter: Fuchs Mehler Weiss & Fritzsche

(56) Entgegenhaltungen:
- EP-A- 0 997 118
- DE-A- 19 826 638
- DE-B- 10 247 397
- DE-B- 10 252 123
- US-A- 6 123 732

## Beschreibung

Die vorliegende Erfindung betrifft einen Übergangsadapter zwischen einem starren transkutanen Implantat, welches intrakorporal in einem Femurstumpf verankerbar ist, und einem Teil eines extrakorporalen orthopädischen Kniegelenks.

Ein derartiges transkutanes Implantat ist bekannt aus der DE 198 26 638 C2. Der darin offenbarte Adapter für ein exoprothetisches Standardteil ist mit einem proximalen Stielteil in einen Röhrenknochenstumpf setzbar, wobei das Stielteil zumindest teilweise mit einer offenmaschigen, dreidimensionalen Raumnetzstruktur bedeckt ist und an seinem distalen Ende mit einer Kopplungseinrichtung für das exoprothetische Standardteil versehen ist. Die offenmaschige, dreidimensionale Raumnetzstruktur, die auch als interkonnektierend bezeichnet wird, ermöglicht ein Ein-, Durch-, Hinter- und Umwachsen von natürlichem Knochenmaterial während der Einheilphase, so dass der Stielteil nach relativ kurzer Zeit jedenfalls im Hinblick auf den Substratfluss im Röhrenknochen integriert wird und eine extrem stabile Sekundärfixation gewährleistet wird. Das distale Ende des Implantates tritt aus dem Gliedmaßenstumpf heraus und bietet eine Ankoppelungsmöglichkeit für ein exoprothetisches Standardteil, beispielsweise ein extrakorporales orthopädisches Gelenk.

Die dadurch erzielte Unmittelbarkeit der Wahrnehmung der externen Belastungen im Skelett des Patienten wird auch als Osteoperzeption bezeichnet. Diese gestattet eine vollkommen andersartige Wahrnehmung des Patienten mit beispielsweise einem oberschenkelamputierten Bein. Bislang war es in einem solchen Fall üblich, dass über den Oberschenkelstumpf ein Prothesenschaft gezogen wurde, an dessen unterem Ende eine Beinprothese angebracht war. Dieser Schaft wird durch einen Justieraufbau und Inaugenscheinnahme in einen willkürlichen Valgitätswinkel verbracht. Während der Gangprobe kann der Oberschenkelschaft durch Veränderung des Valgitätswinkels in engen Grenzen zwischen 0° und 4° verändert werden.

Bei der Anwendung des erwähnten transkutanen Implantates stellt sich das Problem anders dar. Aufgrund der starren Verbindung des Implantates mit dem Femurknochen ist eine "Verstellung" des Valgitätswinkels aufgrund der Weichteilbewegungen des Oberschenkelstumpfes im Prothesenschaft mangels Vorhandensein des letzteren nicht möglich. Dementsprechend liegt der vorliegenden Erfindung die Aufgabe zugrunde, eine Möglichkeit zu schaffen, nach der Implantation eines beschriebenen transkutanen Implantates den natürlichen Valgitätswinkel möglichst genau nachzuempfinden.

Diese Aufgabe wird gelöst durch einen Übergangsadapter der eingangs genannten Art mit den Merkmalen des Anspruchs 1.

Demgemäß weist der Übergangsadapter ein erstes Kopplungsteil zur Verbindung mit dem transkutanen Implantat mit einer Hauptachse und ein zweites Kopplungsteil zur Verbindung mit dem Kniegelenkteil auf, wobei das zweite Kopplungsteil mit dem ersten Kopplungsteil lösbar verbindbar ist und abgewinkelt ist, derart, dass die Hochachse des Kniegelenkteils mit der Hauptachse des ersten Kopplungsteils einen Winkel α im Bereich 4°<α<8° einschließt. Der Hauptachse des ersten Kopplungsteils entspricht dabei die Femurschaftachse.

Der angegebene Winkelbereich ist angemessen bei der Art der Versorgung mit einem transkutanen Implantat. Bislang galt es als ausreichend, bei Beinprothesen der herkömmlichen Art einen Bereich von 0° bis 4° vorzusehen, um den Valgitätswinkel möglichst naturgetreu nachzubilden. Aufgrund der ganz andersartigen Versorgung reicht dieser Winkelbereich im vorliegenden Falle nicht mehr aus und der Winkelbereich muss vergrößert werden. Der einmal ausgewählte Winkel bleibt während des Tragens der Beinprothese stabil. Dies war bislang bei Beinprothesen gemäß dem Stand der Technik nicht gewährleistet, da der Beinstumpf sich im Schaft durch den Hautzug bewegte und so kleinere Winkelveränderungen an der Tagesordnung sind.

Gemäß einer vorteilhaften Weiterbildung ist vorgesehen, dass das zweite Kopplungsteil des Übergangsadapters mit dem Kniegelenkteil einstellbar in einem Winkel β im Bereich 0°<β<4° verbindbar und arretierbar ist. Somit ergibt sich insgesamt ein Winkelbereich von 4° bis 12°, innerhalb dessen der Valgitätswinkel auszuwählen ist. Der jeweilige Übergangsadapter muss patientenindividuell hergestellt werden.

Gemäß einer noch weiteren Ausführungsform ist vorgesehen, dass das erste Kopplungsteil als Hülse mit zylindrischer Außenwandung und eingelassener konischer Klemmhülse ausgebildet ist, in die ein am transkutanen Implantat angebrachter konischer Klemmzapfen setzbar und arretierbar ist. Die Arretierung kann durch eine von unten eingeführte Schraube erzeugt werden, die in eine entsprechende Gewindebohrung in dem konischen Klemmzapfen greift und dort festgezogen werden kann. Durch die erwähnte Maßnahme wird eine besonders feste Verbindung zwischen dem transkutanen Implantat und dem Adapter hergestellt.

Mit den vorstehend wiedergegebenen Ausführungsformen wurden hervorragende Ergebnisse erzielt mit einem physiologischen orthopädischen Kniegelenk, wie es beispielsweise bekannt ist aus der EP 03 585 056 B. Das darin offenbarte Kniegelenk gestattet eine physiologisch nachgeahmte Roll- und Gleitbewegung des Oberteils gegenüber dem Unterteil. Bei Scharniergelenken gestaltet sich die Sache noch etwas komplizierter. Daher ist gemäß einer noch weiteren Ausführungsform vorgesehen, dass die Hochachse des Kniegelenkteils gegenüber der Hauptachse des ersten Kopplungsteils um einen Abstand zwischen 5mm bis 15mm von lateral gesehen nach dorsal versetzt ist. Hierdurch wird die Stand- und Gangsicherheit bei Anwendung von Scharniergelenken erhöht, da dadurch der Kniedrehpunkt möglichst weit nach dorsal verlagert wird, jedoch nur soweit, wie es ein nachempfundener physiologischer Bewegungsablauf zulässt. Vor der Operation wird vom Kniebereich ein Röntgenbild von lateral (seitlicher Strahlengang) angefertigt. Dieses Röntgenbild gibt Aufschluss über den Abstand des Kniedrehpunktes zur Femurschaftachse. Dementsprechend wird ein Übergangadapter gefertigt, mit welchem ein entsprechender Abstand erzeugt werden kann unter Berücksichtigung des Abstandes des Drehpunktes im Scharniergelenk von der Femurschaftachse. Da unterschiedliche Kniescharniergelenke, also technische Kniegelenke, auf dem Markt sind, können diese an das transkutane Implantat durch entsprechende Auswahl und Fertigung eines entsprechenden Übergangsadapters angepasst werden.

Die Erfindung wird anhand eines Ausführungsbeispiels gemäß den Zeichnungsfiguren näher erläutert. Hierbei zeigt:
- Fig. 1: eine schematische Ansicht des Übergangsadapters, verbunden mit einem Kniegelenkteil, von dorsal gesehen,
- Fig. 2: eine ähnliche Ansicht wie Fig. 1, jedoch teilweise geschnitten, und
- Fig. 3: eine Ansicht der Anordnung aus Fig. 1 bzw. Fig. 2, von lateral her gesehen, und
- Fig. 4: eine Ansicht wie in Fig. 3 einer weiteren Ausführungsform.

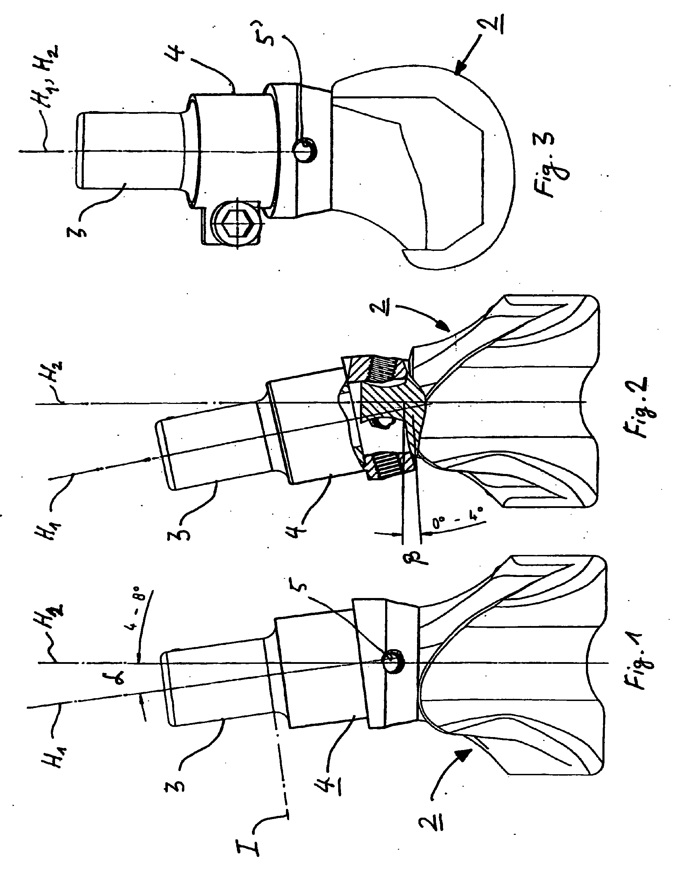

Fig. 1 verschafft einen ersten Überblick. Darin dargestellt ist ein Übergangsadapter mit den Kopplungsteilen 3 und 4. Letzteres ist mit einem Teil des extrakorporalen, orthopädischen Kniegelenks 2 verbunden.

Die Koppelungseinrichtung 3 des Übergangsadapters, die vorliegend als zylindrische Hülse dargestellt ist, wird intrakorporal mit dem transkutanen Implantat (nicht dargestellt) verbunden. Etwa bis zur Höhe der Linie I in Fig. 1 befindet sich der Übergangsadapter nach Anbringung intrakorporal. Es schließt sich ein Übergangsbereich an, in dem sich der Durchmesser des Kopplungsteiles 3 auf den Innendurchmesser des wie eine Buchse ausgebildeten Kopplungsteils 4 vergrößert. In diesem Übergangsbereich kommt vorteilhaft ein subkutanes, intramuskuläres Lager für das starre transkutane Implantat beispielsweise gemäß der DE 102 47 397 zur Anlage.

Das Kopplungsteil 4 ist so ausgebildet, dass es abgewinkelt ist in der Art, dass die Hochachse H₂ des Kniegelenkteils 2 mit der Hauptachse H₁ des ersten Kopplungsteils 3 einen Winkel α einschließt, der im Bereich von 4° bis 8° liegt. Der zutreffende Valgitätswinkel wird patientenindividuell ermittelt und ein entsprechender Übergangsadapter gefertigt.

Fig. 2 zeigt die Anordnung aus Fig. 1, jedoch im Übergangsbereich von dem Kopplungsteil 4 hin zum Kniegelenkteil 2 teilweise geschnitten. Erkennbar ist eine Gewindebohrungen in der Basis des Kopplungsteils 4. Insgesamt sind vier derartige Innengewindebohrungen vorgesehen, in welche Madenschrauben verschraubt werden können, um so die Verbindung zwischen dem Kupplungsteil 4 und dem Kniegelenkteil 2 zu arretieren. Es besteht dabei eine Einstellmöglichkeit im Bereich des Winkels β von 0° bis 4°.

In Verbindung mit dem Winkel α ergeben sich also Einstellungsmöglichkeiten für den Valgitätswinkel im Bereich von 4° bis 12° und damit eine ausreichende Bandbreite, um praktisch alle natürlichen Valgitätswinkel zu erzeugen.

Fig. 3 rundet das Bild ab und verdeutlicht in Verbindung mit den Figuren 1 und 2, in welche Richtung der Valgitätswinkel geneigt ist. Fig. 3 zeigt die Ansichten der Figuren 1 und 2 von lateral. Dargestellt ist hier die Gewindebohrung 5' von insgesamt vier Gewindebohrungen, die gegenüber der Gewindebohrung 5 aus Fig. 1 um 90° versetzt angeordnet ist.

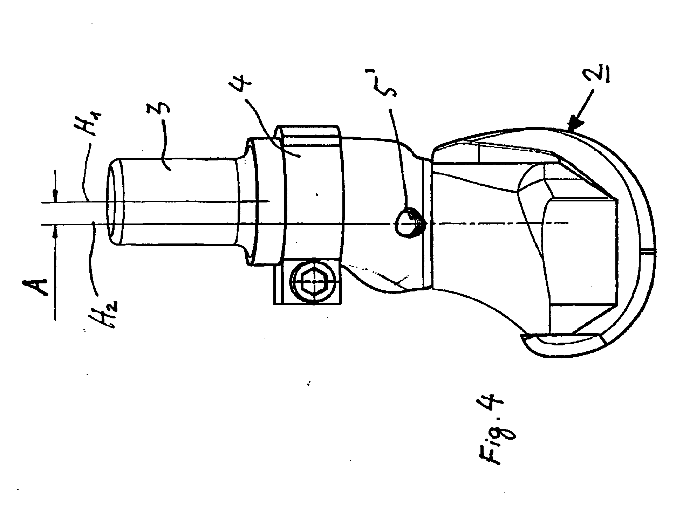

In Fig. 4 ist eine ähnliche Ansicht von lateral auf den Übergangsadapter gemäß einer weiteren Ausführungsform wie in Fig. 3 dargestellt. Der Unterschied zu der Ausführungsform gemäß Fig. 3 ist, dass hier die Hochachse H₂ des Kniegelenkteils 2 gegenüber der Hauptachse H₁ des ersten Kopplungsteils 3 um einen Abstand A von lateral gesehen nach dorsal versetzt ist. A liegt in einem Bereich zwischen 5mm und 15mm. Durch diesen Versatz wird der Kniedrehpunkt eines Knie-Scharniergelenkes möglichst weit nach dorsal verlagert um die Stand- und Gangsicherheit zu erhöhen.

## Patentansprüche

1. Übergangsadapter zwischen einem starren transkutanen Implantat, welches intrakorporal in einem Femurstumpf verankerbar ist, und einem Teil eines extrakorporalen orthopädischen Kniegelenks (2),
**gekennzeichnet durch**
ein erstes Koppelungsteil (3) mit einer Hauptachse (H₁) zur Verbindung mit dem transkutanen Implantat und ein zweites Kopplungsteil (4) zur Verbindung mit dem Kniegelenkteil (2), wobei das zweite Kopplungsteil (4) mit dem ersten Kopplungsteil (3) lösbar verbindbar ist und gegenüber dem ersten Kopplungsteil in einem Winkel α im Bereich 4°<α<8° abgewinkelt ist.

2. Übergangsadapter nach Anspruch 1, bei dem das zweite Kopplungsteil (4) an dem Kniegelenkteil (2) einstellbar in einem Winkel β im Bereich von 0<β<4° verbindbar und arretierbar ist.

3. Übergangsadapter nach Anspruch 1 oder 2, bei dem das erste Kopplungsteil (3) als Hülse mit zylindrischer Außenwandung und eingelassener konischer Klemmhülse ausgebildet ist, in die ein am transkutanen Implantat angebrachter konischer Klemmzapfen setzbar und arretierbar ist.

4. Übergangsadapter nach einem der Ansprüche 1 bis 3, wobei der Übergangsadapter derart gestaltet ist, dass bei Verbindung mit dem Kniegelenkteil (2) eine das Kniegelenkteil (2) aufweisende Hochachse (H₂) gegenüber der Hauptachse (H₁) des ersten Kopplungsteils (3) um einen Abstand (A) zwischen 5mm bis 15mm von lateral gesehen nach dorsal versetzt ist.

## Claims

1. Transition adapter between a rigid transcutaneous implant, which can be anchored intracorporeally in a femoral stump, and a part of an extracorporeal orthopaedic knee joint (2), **characterised by** a first coupling part (3) with a main axis (H₁) for connection to the transcutaneous implant and a second coupling part (4) for connection to the knee joint part (2), wherein the second coupling part (4) can be connected releasably to the first coupling part (3) and with respect to the first coupling part is bent at an angle α in the range 4°<α<8°.

2. Transition adapter according to claim 1, in which the second coupling part (4) can be connected and locked on the knee joint part (2) adjustably at an angle β in the range from 0<β<4°.

3. Transition adapter according to claim 1 or 2, in which the first coupling part (3) is designed as a sleeve with cylindrical outer wall and countersunk conical clamping sleeve, into which a conical clamping pin attached to the transcutaneous implant can be placed and locked.

4. Transition adapter according to one of claims 1 to 3, wherein the transition adapter is designed such that when connecting to the knee joint part (2), a vertical axis (H₂) having the knee joint part (2) is displaced with respect to the main axis (H₁) of the first coupling part (3) by a distance (A) between 5 mm to 15 mm seen laterally to dorsally.

## Revendications

1. Adaptateur de transition entre un implant transcutané rigide, que l'on peut ancrer dans le corps dans un moignon de fémur et un élément d'une rotule (2) extracorporelle orthopédique,
**caractérisé par**
un premier élément de couplage (3), avec un axe principal (H₁), destiné à l'assemblage avec l'implant transcutané et un deuxième élément de couplage (4) destiné à l'assemblage avec l'élément de rotule (2), le deuxième élément de couplage (4) pouvant être relié de façon amovible avec le premier élément de couplage (3) et étant coudé d'un angle α dans une fourchette de 4° < α < 8°.

2. Adaptateur de transition selon la revendication 1, sur lequel le deuxième élément de couplage (4) peut être relié et bloqué sur l'élément de rotule (2), de façon réglable sous un angle β dans la fourchette de 0 < β < 4°.

3. Adaptateur de transition selon la revendication 1 ou 2, sur lequel le premier élément de couplage (3) est conçu en tant qu'une douille avec une paroi extérieure cylindrique et une douille de serrage conique encastrée, sur laquelle on peut poser et bloquer un tenon de serrage conique monté sur l'implant transcutané.

4. Adaptateur de transition selon l'une quelconque des revendications 1 à 3, l'adaptateur de transition étant conçu de façon telle que, lors de la liaison avec l'élément de rotule (2), un axe vertical (H₂) comportant l'élément de rotule (2) est déporté d'une distance (A) entre 5 mm et 15 mm, vu latéralement vers dorsalement, par rapport à l'axe principal (H₁) du premier élément de couplage (3).
